# EUROPEAN PATENT SPECIFICATION

(11) **EP 1 617 839 B1**
(45) Date of publication and mention of the grant of the patent: **18.06.2008**
(21) Application number: 04730286.4
(22) Date of filing: 29.04.2004
(51) Int. Cl.: A61K 31/397, A61P 43/00, A61P 25/18, A61P 25/28, A61P 25/00, A61P 1/00, A61P 25/34, A61P 3/04, A61P 3/10

(54) **THE USE OF AZETIDINECARBOXAMIDE DERIVATIVES IN THERAPY**
VERWENDUNG VON AZETIDINCARBOXAMIDDERIVATEN IN THERAPIE
UTILISATION DE DERIVES D'AZETIDINECARBOXAMIDE A DES FINS THERAPEUTIQUES

(30) Priority: 01.05.2003 GB 0310057
(43) Date of publication of application: 25.01.2006
(73) Proprietor: VERNALIS RESEARCH LIMITED, Winnersh, Wokingham RG41 5UA (GB)
(72) Inventor: DAVIDSON, James E.P., Vernalis Research Ltd., Winnersh RG415UA (GB); ADAMS, David R., Vernalis Research Ltd., Winnersh RG41 5UA (GB); BICKERDIKE, Michael J, Vernalis Research Ltd., Winnersh RG41 5UA (GB); FLETCHER, Allan, Vernalis Research Ltd., Winnersh RG41 5UA (GB)
(74) Representative: Walls, Alan James
(86) International application number: PCT/GB2004/001812
(87) International publication number: WO 2004/096209

(56) References cited:
- WO-A-01/07023
- WO-A-99/37612
- FR-A- 2 805 810
- FR-A- 2 805 817
- "The Merck Manual, Seventeenth Edition" 1999, MERCK RESEARCH LABORATORIES Dementia * page 1393 - page 1395 *

## Description

The present invention relates primarily to the use of azetidine-1-carboxamides in the treatment of disorders mediated by the cannabinoid CB₁ receptor, particularly to the treatment of obesity and other eating disorders associated with excessive food intake.

It has been recognised that obesity is a disease process influenced by environmental factors in which the traditional weight loss methods of dieting and exercise need to be supplemented by therapeutic products (S. Parker, "Obesity: Trends and Treatments", Scrip Reports, PJB Publications Ltd, 1996).

Whether someone is classified as overweight or obese is generally determined on the basis of their body mass index (BMI) which is calculated by dividing body weight (kg) by height squared (m²). Thus, the units of BMI are kg/m² and it is possible to calculate the BMI range associated with minimum mortality in each decade of life. Overweight is defined as a BMI in the range 25-30 kg/m², and obesity as a BMI greater than 30 kg/m². There are problems with this definition in that it does not take into account the proportion of body mass that is muscle in relation to fat (adipose tissue). To account for this, obesity can also be defined on the basis of body fat content: greater than 25% and 30% in males and females, respectively.

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

Compounds marketed as anti-obesity agents include Orlistat (Reductil®) and Sibutramine. Orlistat (a lipase inhibitor) inhibits fat absorption directly and tends to produce a high incidence of unpleasant (though relatively harmless) side-effects such as diarrhoea. Sibutramine (a mixed 5-HT/noradrenaline reuptake inhibitor) can increase blood pressure and heart rate in some patients. The serotonin releaser/reuptake inhibitors fenfluramine (Pondimin®) and dexfenfluramine (Redux^{™}) have been reported to decrease food intake and body weight over a prolonged period (greater than 6 months). However, both products were withdrawn after reports of preliminary evidence of heart valve abnormalities associated with their use. There is therefore a need for the development of a safer anti-obesity agent.

There now exists extensive pre-clinical and clinical data supporting the use of CB₁ receptor antagonists / inverse agonists for the treatment of obesity.

Preparations of marijuana *(Cannabis sativa)* have been used for over 5000 years for both medicinal and recreational purposes. The major psychoactive ingredient of marijuana has been identified as Δ⁹-tetrahydrocannabinol (Δ⁹-THC), one of a member of over 60 related cannabinoid compounds isolated from this plant. It has been demonstrated that Δ⁹-THC exerts its effects via agonist interaction with cannabinoid (CB) receptors. So far, two cannabinoid receptor subtypes have been characterised (CB₁ and CB₂). The CB₁ receptor subtype is found predominantly in the central nervous system, and to a lesser extent in the peripheral nervous system and various peripheral organs. The CB₂ receptor subtype is found predominantly in lymphoid tissues and cells. To date, three endogenous agonists (endocannabinoids) have been identified which interact with both CB₁ and CB₂ receptors (anandamide, 2-arachidonyl glycerol and noladin ether).

Genetically obese rats and mice exhibit markedly elevated endocannabinoid levels in brain regions associated with ingestive behaviour (Di Marzo et al. 2001 Nature 410: 822 - 825). Furthermore, increased levels of endocannabinoids are observed upon the fasting of normal, lean animals (Kirkham et al., British Journal of Pharmacology, 2002, 136(4), 550-557). Exogenous application of endocannabinoids leads to the same physiological effects observed with Δ⁹-THC treatment, including appetite stimulation (Jamshida et al., British Journal of Pharmacology, 2001, 134: 1151-1154), analgesia, hypolocomotion, hypothermia, and catalepsy.

CB₁ (CB₁^{-/-}) and CB₂ (CB₂^{-/-}) receptor knockout mice have been used to elucidate the specific role of the two cannabinoid receptor subtypes. Furthermore, for ligands such as Δ⁹-THC which act as agonists at both receptors, these mice have allowed identification of which receptor subtype is mediating specific physiological effects. CB₁^{-/-}, but not CB₂^{-/-}, mice are resistant to the behavioural effects of agonists such as Δ⁹-THC. CB₁^{-/-} animals have also been shown to be resistant to both the body weight gain associated with chronic high fat diet exposure, and the appetite-stimulating effects of acute food deprivation.

These findings suggest a clear role for both endogenous and exogenous cannabinoid receptor agonists in increasing food intake and body weight via selective activation of the CB₁ receptor subtype.

The therapeutic potential for cannabinoid receptor ligands has been extensively reviewed (Exp. Opin. Ther. Pat. 1998, 8, 301-313; Exp. Opin. Ther. Pat. 2000, 10, 1529-1538; Trends in Pharm. Sci. 2000, 21, 218-224; Exp. Opin. Ther. Pat. 2002, 12(10), 1475-1489).

At least one compound (SR-141716A) characterised as a CB₁ receptor antagonist / inverse agonist is known to be in clinical trials for the treatment of obesity.

WO 00/15609, WO 01/64632, WO 01/64633 and WO 01/64634 disclose azetidine derivatives as CB₁ receptor antagonists. WO 02/28346 discloses the association of an azetidine derivative as a CB₁ receptor antagonist, and sibutramine, for the treatment of obesity.

There remains a medical need for low molecular weight CB₁ receptor antagonists / inverse agonists with pharmacokinetic and pharmacodynamic properties making them suitable for use as pharmaceutical agents. There also remains a medical need for new treatments of disorders mediated by the CB₁ receptor, particularly eating disorders, and particularly obesity. The object of the present invention is to provide such pharmaceutical agents and treatments.

It has now been found that certain azetidine-1-carboxamides show unexpected efficacy as anti-obesity agents. These compounds were previously described in WO-A-99/37612 for the treatment of anxiety and epilepsy. These azetidine-1-carboxamides have been shown to selectively bind to the CB₁ receptor subtype with high affinity. Such compounds have been shown to dose-dependently block the effects of an exogenously applied cannabinoid receptor agonist (*eg*Δ⁹-THC) in mice. Furthermore, such compounds have been shown to reduce food intake and body weight gain in both rat and mouse models of feeding behaviour.

According to the present invention, there is provided use of a compound of formula (I) 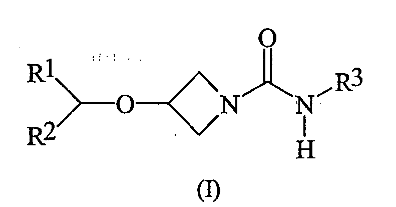 wherein:
R¹ is aryl;
R² is H, alkyl or aryl; and
R³ is hydrogen or alkyl;
or a pharmaceutically acceptable salt thereof, in the manufacture of a medicament for the treatment of a disorder mediated by CB₁ receptors, such as defined in the claims.

The active compounds of formula (I) are antagonists and/or inverse agonists at the cannabinoid-1 (CB₁) receptor and are useful for the treatment, prevention and suppression of diseases mediated by the CB₁ receptor. The invention is concerned with the use of these compounds to selectively antagonise the CB₁ receptor and, as such, in the treatment of obesity and other disorders.

Reference in the present specification to an "alkyl" group means a branched or unbranched, cyclic or acyclic, saturated or unsaturated (e.g. alkenyl (including allyl) or alkynyl (including propargyl)) hydrocarbyl radical. Where cyclic or acyclic the alkyl group is preferably C₁ to C₁₂, more preferably C₁ to C₈ (such as methyl, ethyl, propyl, isopropyl, butyl, isobutyl, tert-butyl, sec-butyl, pentyl, isopentyl, hexyl, heptyl, octyl). It will be appreciated therefore that the term "alkyl" as used herein includes alkyl (branched or unbranched), alkenyl (branched or unbranched), alkynyl (branched or unbranched), cycloalkyl, cycloalkenyl and cycloalkynyl. A cyclic alkyl group may also be a mono-bridged or multi-bridged cyclic alkyl group. In a preferred embodiment, a cyclic alkyl group is preferably C₃ to C₁₂, more preferably C₅ to C₈ and an acyclic alkyl group is preferably C₁ to C₁₀, more preferably C₁ to C₆, more preferably methyl, ethyl, propyl (n-propyl or isopropyl), butyl (n-butyl, isobutyl, tertiarybutyl or sec-butyl) or pentyl (including n-pentyl and iso-pentyl), more preferably methyl.

Reference in the present specification to an "aryl" group means a mono or bicyclic aromatic group, such as phenyl or naphthyl, and preferably a mono-cyclic aromatic group.

Reference in the present specification to a "heteroaryl" group means an aromatic group containing one or more heteroatoms, preferably 1, 2 or 3 heteroatoms, preferably 1 or 2 heteroatoms. Preferably the heteroatoms are selected from O, S and N, preferably from O and N. Preferably the heteroaryl group comprises 5 or 6-membered ring systems. The heteroaryl group is preferably a monocyclic or bicyclic ring system, preferably monocyclic. Examples include thienyl, furanyl, pyrrolyl, imidazolyl, pyrazolyl, thiazolyl, isothiazolyl, oxazolyl, isoxazolyl, pyridyl, pyrazinyl, pyrimidinyl, quinolinyl, isoquinolinyl, benzofuranyl and isobenzofuryl.

Reference in the present specification to a non-aromatic heterocylic group is to a saturated or partially unsaturated 4, 5, 6 or 7-membered ring containing 1, 2 or 3 heteroatoms selected from N, O and S, preferably 1 or 2 heteroatoms, preferably selected from N and O. Examples include piperidinyl, morpholinyl, piperazinyl and pyrrolidinyl.

The alkyl and aryl groups may be substituted or unsubstituted. In one embodiment, only the alkyl and aryl groups defined herein as R¹ to R³ and R⁹ to R¹³ may be substituted. Where substituted, there will generally be 1 to 3 substituents present, preferably 1 or 2 substituents. Substituents may include:
carbon containing groups such as alkyl aryl, arylalkyl (e.g. substituted and unsubstituted phenyl, substituted and unsubstituted benzyl);
halogen atoms and halogen containing groups such as haloalkyl (e.g. trifluoromethyl);
oxygen containing groups such as alcohols (e.g. hydroxy, hydroxyalkyl, (aryl)(hydroxy)alkyl), ethers (e.g. alkoxy, alkoxyalkyl, aryloxyalkyl), aldehydes (e.g. carboxaldehyde), ketones (e.g. alkylcarbonyl, alkylcarbonylalkyl, arylcarbonyl, arylalkylcarbonyl, arylcarbonylalkyl), acids (e.g. carboxy, carboxyalkyl), acid derivatives such as esters (e.g. alkoxycarbonyl, alkoxycarbonylalkyl, alkylcarbonyloxy, alkylcarbonyloxyalkyl) and amides (e.g. aminocarbonyl, mono- or dialkylaminocarbonyl, aminocarbonylalkyl, mono- or dialkylaminocarbonylalkyl, arylaminocarbonyl);
nitrogen containing groups such as amines (e.g. amino, mono- or dialkylamino, aminoalkyl, mono- or dialkylaminoalkyl), azides, nitriles (e.g. cyano, cyanoalkyl), nitro;
sulphur containing groups such as thiols, thioethers, sulphoxides and sulphones (e.g. alkylthio, alkylsulfinyl, alkylsufonyl, alkylthioallcyl, alkylsulfinylalkyl,
alkylsulfonylalkyl, arylthio, arylsulfinyl, arylsulfonyl, arylthioalkyl, arylsulfinylalkyl, arylsulfonylalkyl);
and heterocyclic groups containing one or more, preferably one, heteroatom, (e.g. thienyl, furanyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, piperazinyl, morpholinyl, thionaphthyl, benzofuranyl, isobenzofuryl, indolyl, oxyindolyl, isoindolyl, indazolyl, indolinyl, 7-azaindolyl, isoindazolyl, benzopyranyl, coumarinyl, isocoumarinyl, quinolyl, isoquinolyl, naphthridinyl, cinnolinyl, quinazolinyl, pyridopyridyl, benzoxazinyl, quinoxadinyl, chromenyl, chromanyl, isochromanyl and carbolinyl).

Where an aryl group is phenyl, the phenyl may be substituted by adjacent substituents forming a 5 or 6 membered saturated ring optionally containing 1 or 2 heteroatoms, preferably selected from N, O and S, preferably from N and O. Where the saturated ring contains 2 nitrogen atoms, the ring is preferably a 6-membered ring. Where the saturated ring contains 2 oxygen atoms, the ring may be a 5- or 6-membered ring. Examples include 2,3-dihydrobenzo[b]furan-7-yl, 2,3-dihydrobenzo[b]thiophen-6-yl, 1,2,3,4-tetrahydronaphthalen-5-yl, 2,3-dihydrobenzo[1,4]dioxin-6-yl and 1,2,3,4-tetrahydroisoquinolin-8-yl.

Preferred substituents include alkyl (including haloalkyl), alkoxy (including haloalkoxy), aryl, nitrile or halo. Preferred halogen-containing groups include trifluoromethyl.

As used herein, the term "alkoxy" means alkyl-O- and "alkoyl" means alkyl-CO-.

As used herein, the term "halogen" means a fluorine, chlorine, bromine or iodine radical, preferably a fluorine or chlorine radical.

The compounds of formula (I) may exist in a number of diastereomeric and/or enantiomeric forms. Unless otherwise stated, reference in the present specification to "a compound of formula (I)" is a reference to all stereoisomeric forms of the compound and includes a reference to the unseparated stereoisomers in a mixture, racemic or non-racemic, and to each stereoisomer in its pure form.

In the compounds of formula (I), preferably R¹ is substituted or unsubstituted phenyl or naphthyl (preferably phenyl), more preferably R¹ is a substituted phenyl or naphthyl (preferably phenyl), more preferably R¹ is a phenyl or naphthyl (preferably phenyl), having 1 to 3 substituents and most preferably R¹ is a phenyl or naphthyl (preferably phenyl), having 1 or 2 substituents. Preferred substituents include alkyl, halo, halogen-containing groups such as haloalkyl (particularly halomethyl, such as trifluoromethyl), thioalkyl, alkoxy, alkylsulfonyl, and mono- or di-alkylaminocarbonyl. Particularly preferred substituents are alkyl, halo and halogen-containing groups such as haloalkyl (particularly halomethyl, such as trifluoromethyl); more preferably halo and halogen-containing groups such as haloalkyl (particularly halomethyl, such as trifluoromethyl).

In one embodiment of the invention, R² is aryl, preferably substituted or unsubstituted phenyl, more preferably substituted phenyl, more preferably phenyl having 1 to 3 substituents and most preferably phenyl having 1 or 2 substituents. Preferred substituents include alkyl, halo, halogen-containing groups such as haloalkyl (particularly halomethyl, such as trifluoromethyl), thioalkyl, alkoxy, alkylsulfonyl, and mono- or di-alkylaminocarbonyl. Particularly preferred substituents are alkyl, halo and halogen-containing groups such as haloalkyl (particularly halomethyl, such as trifluoromethyl); more preferably halo and halogen-containing groups such as haloalkyl (particularly halomethyl, such as trifluoromethyl).

In an alternative embodiment, R² is H or alkyl (cyclic or acyclic).

In a preferred embodiment, R³ is alkyl, and preferably selected from alkenyl, alkynyl, hydroxyalkyl, alkoxyalkyl or unsubstituted saturated cyclic or acyclic hydrocarbyl. Preferably R³ is acyclic hydrocarbyl, preferably lower alkyl, and in one embodiment is substituted. One or two substituent groups may be present, preferably one substituent group. Preferred substituents are those referred to hereinabove, particularly hydroxy, alkoxy, thioalkyl, amino, mono- and dialkyl amino, alkoxycarbonyl, aryl (preferably phenyl), and heterocyclic groups including both heteroaryl and non-aromatic heterocyclic groups. Where R³ is an acyclic alkyl group, it may be substituted by a cyclic alkyl group; and where R³ is a cyclic alkyl group it may be substituted by an acyclic alkyl group. Where the substituent group is heteroaryl, the heteroaryl preferably is a 5- or 6-membered ring containing one or more N, O or S atoms, and preferred groups include thiophenyl, furanyl, isoxazolyl, thiazolyl and benzothiophenyl. Other preferred substituent groups include dihydrobenzofuranyl, dihydrobenzodioxinyl, tetrahydrofuranyl, pyrrolidinyl, oxopyrrolindyl and benzodioxolyl.

In one embodiment, R³ is selected from :

-(CHR⁹)ₙ(CH₂)ₘCR¹⁰R¹¹R¹²

wherein n is 0 or 1;
m is 0,1,2 or 3;
R⁹, R¹⁰, R¹¹ and R¹² are selected from hydrogen, alkyl (preferably lower alkyl), hydroxy, alkoxy (preferably lower alkoxy), thioalkyl (preferably thio lower alkyl), amino, mono- and di-alkyl amino (preferably lower alkyl amino), alkoxycarbonyl (preferably lower alkoxy carbonyl) and R¹³;
wherein R¹³ is selected from aryl, heteroaryl and non-aromatic heterocyclic optionally substituted by one or more (preferably 1 or 2, preferably 1) groups preferably selected from alkyl (preferably lower alkyl, preferably methyl), halogen (preferably fluoro, chloro and bromo), alkoxy (preferably lower alkoxy, preferably methoxy), oxo, aryl, heteroaryl and non-aromatic heterocycle.

Preferably, m is 0 or 1 or 2, preferably 0 or 1, and preferably 0.

Preferably, n is 0.

In one embodiment, at least one and more preferably two of R¹⁰, R¹¹ and R¹² are selected from hydrogen. In a further embodiment, at least one and more preferably at least two of R¹⁰, R¹¹ and R¹² are selected from methyl.

In a further embodiment, R³ is selected from cyclic alkyl, including cyclopentyl, cyclohexyl, norbornanyl and adamantyl, preferably cyclopentyl and cyclohexyl.

Preferred R³ groups are tertiary butyl, sec-butyl, isobutyl, isopropyl, n-propyl and ethyl, particularly tertiary butyl, isobutyl, sec-butyl and isopropyl, and particularly tertiary butyl.

Compounds of formula (I) include:

| **R¹** | **R²** | **R³** |
|---|---|---|
| 4-Cl-C₆H₄ | 4-Cl-C₆H₄ | Allyl |
| 4-Cl-C₆H₄ | 4-Cl-C₆H₄ | 2-Hydroxypropyl |

The diseases and disorders to which the present invention is directed are:
psychosis, schizophrenia, attention deficit disorder, gastrointestinal disorders (such as dysfunction of gastrointestinal motility or diarrhoea), smoking cessation, obesity and other eating disorders associated with excessive food intake (including bulimia and compulsive eating disorder) and associated health complications including non-insulin dependent diabetes mellitus. The present invention is particularly directed to obesity and other eating disorders associated with excessive food intake and associated health complications including non-insulin dependent diabetes mellitus, and particularly to obesity and other eating disorders associated with excessive food intake, and especially to obesity.

In an alternative embodiment, the present invention is directed to smoking cessation and the facilitation thereof.

In a further alternative embodiment, the present invention is directed to gastrointestinal disorders (such as dysfunction of gastrointestinal motility or diarrhoea).

The present invention may be employed in respect of a human or animal subject, more preferably a mammal, more preferably a human subject.

As used herein, the term "treatment" as used herein includes prophylactic treatment.

As used herein, the term "pharmaceutically acceptable salt" means any pharmaceutically acceptable salt of the compound of formula (I). Salts may be prepared from pharmaceutically acceptable non-toxic acids and bases including inorganic and organic acids and bases. Such acids include acetic, benzenesulfonic, benzoic, camphorsulfonic, citric, ethenesulfonic, dichloroacetic, furnaric, gluconic, glutamic, hippuric, hydrobromic, hydrochloric, isethionic, lactic, maleic, malic, mandelic, methanesulfonic, mucic, nitric, pamoic, pantothenic, phosphoric, succinic, sulfuric, tartaric, oxalic, p-toluenesulfonic and the like. Particularly preferred are hydrochloric, hydrobromic, phosphoric, sulfuric and methanesulfonic acids, and most particularly preferred is the methanesulfonate salt. Acceptable base salts include alkali metal (e.g. sodium, potassium), alkaline earth metal (e.g. calcium, magnesium) and aluminium salts.

The compound of formula (I) may be used in combination with one or more additional drugs useful in the treatment of the disorders mentioned above, the components being in the same formulation or in separate formulations for administration simultaneously or sequentially.

Compounds of formula (I) may be prepared according to Reaction Scheme 1 (where P is a nitrogen protecting group). R¹, R², and R³ are as previously defined. The ether (IV) may be formed by reaction of the azetidinol (II) either with an arylalkanol (III, X = OH) and diethylazo dicarboxylate and triphenyl phosphine or with an arylalkyl chloride, bromide, iodide, mesylate or tosylate (III, X = Cl, Br, I, mesylate, tosylate) and a strong base such as sodium hydride. Formation of the azetidine (V) may be achieved by reaction of (IV) with a suitable nitrogen deprotection agent. For example, if P is a diphenylmethyl group, then deprotection may be carried out by treatment with 1-chloroethyl chloroformate followed by methanol. The urea (I) is formed by reaction of azetidine (V) with an N-alkylisocyanate or an N-alkylcarbamoyl chloride and a base such as triethylamine or potassium carbonate. Alternatively, the urea may be prepared directly from the azetidine (IV) without isolation of an intermediate such as the secondary amine (V). For example, when P is a diphenylmethyl group, azetidine (TV) may be treated with phosgene followed by amine R³NH₂ to give urea (I) directly.

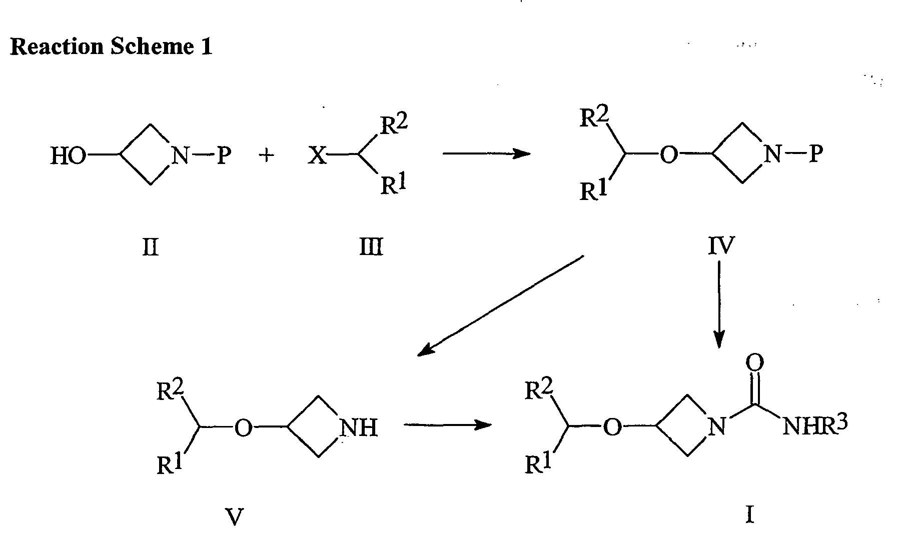

Compounds of formula (I) where R¹ and R² are aryl may be prepared according to Reaction Scheme 2 (where P is a nitrogen protecting group). R¹, R², and R³ are as previously described. The ether (IV) may be formed by reaction of the azetidinol (II) with a benzhydrol (III, X = OH) with removal of water (for example azeotropic removal of water under standard Dean-Stark conditions). The ether (IV) may also be formed by reaction of the azetidinol (II) with a benzhydryl group substituted with a suitable leaving-group (III, X = Cl, Br, I, mesylate, tosylate) and a strong base such as sodium hydride. Formation of the azetidine (V) may be achieved by reaction of (IV) with a suitable nitrogen deprotection agent. For example, if P is a benzhydryl group, then deprotection may be carried out by treatment with 1-chloroethyl chloroformate followed by treatment with methanol. The deprotected azetidine (V) can be isolated directly as the hydrochloride salt or, upon basification, as the free-base. The urea (I) can be formed by reaction of azetidine (V) with an N-alkyl isocyanate, or an N-alkyl carbamoyl chloride and a base such as triethylamine or potassium carbonate. Alternatively, the urea may be prepared directly from the protected azetidine (IV) without isolation of the intermediate azetidine (V). For example, when P is a benzhydryl group, azetidine (IV) may be treated with phosgene followed by an amine, R³NH₂, to give urea (I) directly. Azetidine (V) may also be converted to the corresponding carbamoyl chloride (VI) by treatment with, for example, triphosgene. This intermediate carbamoyl chloride (VI) may be reacted with an amine, R³NH₂, to give the urea (I).

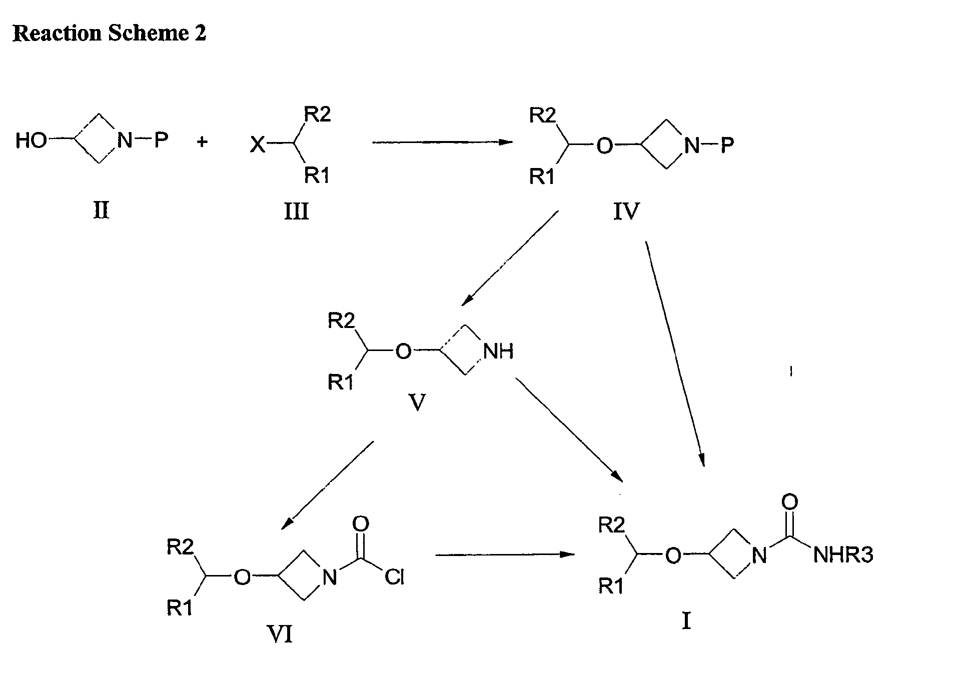

The invention further provides a pharmaceutical composition comprising an effective amount of the compound of formula (I) in combination with a pharmaceutically acceptable carrier or excipient and a method of making such a composition comprising combining an effective amount of the compound of formula (I) with a pharmaceutically acceptable carrier or excipient.

To further increase efficacy, the composition may contain components such as dextrans or cyclodextrins or ether derivatives thereof, which aid stability and dispersion, and decrease metabolism of the active ingredient.

For compositions in which the pharmaceutically acceptable carrier comprises a cyclodextrin or an ether derivative thereof, the active ingredient is intimately mixed with an aqueous solution of the cyclodextrin or ether derivative thereof, with optional addition of further pharmaceutically acceptable ingredients before, during or after said mixing. The thus obtained solution is optionally lyophilized, and the lyophilized residue is optionally reconstituted with water.

In an embodiment of the present invention, the composition further comprises a buffer system, an isotonizing agent and water.

Compounds of formula (I) may be administered in a form suitable for oral use, for example a tablet, capsule, aqueous or oily solution, suspension or emulsion; for topical use including transmucosal and transdermal use, for example a cream, ointment, gel, aqueous or oil solution or suspension, salve, patch or plaster; for nasal use, for a example a snuff, nasal spray or nasal drops; for vaginal or rectal use, for example a suppository; for administration by inhalation, for example a finely divided powder or a liquid aerosol; for sub-lingual or buccal use, for example a tablet or capsule; or for parenteral use (including intravenous, subcutaneous, intramuscular, intravascular or infusion), for example a sterile aqueous or oil solution or suspension. In general the above compositions may be prepared in a conventional manner using conventional excipients, using standard techniques well known to those skilled in the art of pharmacy. Preferably, the compound is administered orally.

For oral administration, the compounds of formula (I) will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil. Alternatively, the active ingredient may be mixed with excipients, surfactants or solubilising agents such as Labrafil®, Labrasol® or Miglyol®, or appropriate mixtures thereof.

For intramuscular, intraperitoneal, subcutaneous and intravenous use, the compounds of formula (I) will generally be provided in sterile aqueous solutions or suspensions, buffered to an appropriate pH and isotonicity. Suitable aqueous vehicles include Ringer's solution and isotonic sodium chloride. Aqueous suspensions may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

It will be appreciated that the dosage levels used may vary over quite a wide range depending upon the compound used, the severity of the symptoms exhibited by the patient and the patient's body weight.

The invention will now be described in detail with reference to the following pharmacological examples.

### EXAMPLES

### Synthetic Examples

### Preparation of 1-(Diphenylmethyl)-3-azetidinol

This compound was prepared according to the method of Anderson and Lok (J. Org. Chem., 1972, 37, 3953, the disclosure of which is incorporated herein by reference), m.p. 111-112 °C (lit. m.p. 113 °C).

### Preparation of 3-(4-Chlorobenzyloxy)-1-(diphenylmethyl) azetidine (1)

A solution of 1-diphenylmethyl-3-azetidinol (25 mmol) in DMF (100 mL) was added at 0 °C to a suspension of NaH (60% disp.in oil, 30 mmol) in DMF (50 mL). The reaction mixture was stirred at room temperature for 1h, then 4-chlorobenzylchloride (25 mmol) was added dropwise at 0 °C and the reaction mixture stirred at room temperature for 3 h. The reaction was quenched with water and extracted with ethyl acetate (3 x 50 mL), the extracts were washed with water and brine, dried (MgSO₄) and concentrated *in vacuo.* The residue was purified by chromatography [SiO₂; hexane-ethyl acetate (9:1)] to yield the product as a yellow oil (7.3 g, 80%). The material was used in the next step without further purification.

### Example 1. 3-(4-Chlorobenzyloxy)-N-(2-propenyl)azetidine-1-carboxamide (2)

Phosgene solution (1.75-M in toluene, 24 mmol) was added at 0°C to a solution of compound (1) (20 mmol) in CH₂Cl₂ (40 mL). The reaction mixture was stirred at room temperature for 90 min, concentrated *in vacuo,* then redissolved in CH₂Cl₂ (40 mL) and treated with allylamine (42 mmol) at 0°C. The reaction was stirred for 4 h at room temperature, then water (40 mL) was added and the layers were separated. The aqueous layer was extracted with further CH₂Cl₂ (2 x 40 mL). The organic layers were washed with dilute HCl (20 mmol) and brine, dried (MgSO₄) and concentrated *in vacuo.* The residue was triturated using diethyl ether to give the product (2) as a crystalline solid (3.5 g, 60%), m.p. 110-111 °C. Found: C, 59.84; H, 6.11; N, 9.98. C₁₄H₁₇ClN₂O₂ requires: C, 59.89; H, 9.6.10; N, 9.97%.

### Preparation of 3-(3,4-Dichlorobenzyloxy)-1-(diphenylmethyl) azetidine (3)

This material was prepared from 1-diphenylmethyl-3-azetidinol (6.0 g) and alpha,3,4-trichlorotoluene using the procedure described for compound (1) (yield 92%).

### Example 2. 3-(3,4-Dichlorobenzyloxy)-N-(2-propenyl)azetidine-l-carboxamide (4)

This material was prepared from compound (3) (9.2 g) using the procedure described for compound (2) (yield 75%), m.p. 88-89 °C. Found: C, 53.43; H, 5.18; N, 8.85, C₁₄H₁₆Cl₂N₂O₂ requires C, 53.35; H, 5.12; N, 8.88%.

### Preparation of 3-(3-(Trifluoromethyl)benzyloxy)-1-(diphenylmethyl)azetidine (5)

This material was prepared from 1-diphenylmethyl-3-azetidinol (5 g) and alpha'-bromo-alpha,alpha,alpha-trifluoro-nz-xylene using the procedure described for compound (1) (yield 91%).

### Example 3. 3-(3-(Trffluoromethyl)benzyloxy)-N-(2-propenyl)azetidine-l-carboxamide (6)

This material was prepared from compound (5) (7.5 g) using the procedure described for compound (1) (yield 64%), m.p. 108°C. Found: C, 57.29; H, 5.44; N, 8.87, C₁₅H₁₇F₃N₂O₂ requires C, 57.32; H, 5.45; N, 8.91%.

### Preparation of 3-(4-(Trifluoromethyl)benzyloxy)-1-(diphenylmethyl)azetidine (7)

This material was prepared from 1-diphenylinethyl-3-azetidinol (6.0 g) and a'-bromo-a,a,a-trifluoro-p-xylene using the procedure described for compound (1) (yield 77%).

### Example 4. 3-(4-(Trifluoromethyl)benzyloxy)-N-(2-propenyl)azetidine-1-carboxamide (8)

This material was prepared from compound (7) (7.7 g) using the procedure described for compound (2) (yield 72%), m.p. 120 °C. Found: C, 57.27; H, 5.45; N, 8.86. C₁₅H₁₇F₃N₂O₂ requries C, 57.32; H, 5.45, N, 8.91 %.

### Preparation of 3-(4-Fluorobenzyloxy)-1-(diphenylmethyl) azetidine (9)

This material was prepared from 1-diphenylmethyl-3-azetidinol (6.0 g) and 4-fluorobenzyl bromide using the procedure described for compound (1) (yield 83%).

### Example 5. 3-(4-Fluorobenzyloxy)-N-(2-propenyl)azetidine-1-carboxamide (10)

This material was prepared from compound (9) using the procedure described for compound (2), m.p. 97-99 °C. Found: C, 63.57; H, 6.59; N, 10.66. C₁₄H₁₇CIN₂O₂ requires C, 63.62; H, 6.48; N, 10.59.

### Preparation of 3-(bis-(4-chlorophenyl)methoxy-1-diphenylmethyl)azetidine (11)

A solution of 4,4'-dichlorobenzhydrol (25 mmol), p-toluenesulfonic acid (18.4 mmol) and 1-(diphenylmethyl)-3-azetidinol (8.4 mmol) in benzene (100 mL) was heated under reflux in a Dean-Stark apparatus for 3h. The solution was cooled, washed with sodium hydrogen carbonate (saturated aqueous solution, 100 mL), dried (MgSO₄) and concentrated *in vacuo.* The residue was purified by chromatography [SiO₂; hexane-diethyl ether (5:1)] to yield the product (11) as a thick oil that crystallized on standing (2.4g, 62%).

### Example 6. 3-(Bis(4-chlorophenyl)methoxy)-N-(2-propenyl)azetidine-1-carboxamide (12)

This material was prepared from compound (11) using the procedure described for compound (2) (yield 17%) as a crystalline solid. Found: C, 56.38; H, 5.10; N, 6.51. C₂₀H₂₀CI₂N-₂O₂.2H₂O requires: C, 56.21; H, 5.66; N, 6.56%.

### Example 7. Preparation of (R)-3-(Bis(4-chlorophenyl)methoxy)-N-(2-hydroxypropyl)azetidine-1-carboxamide (13)

This material was prepared from compound (11) and (*R*)-(-)-1-amino-2-propanol using the procedure described for compound (2) (yield 57%) as a crystalline solid. Found: C, 58.74; H, 5.42; N, 6.84. C₂₀H₂₂Cl₂N₂O₃ requires: C, 58.69; H, 5.42; N, 6.84%.

### Example 8.3-(3-Trifluoromethyl)benzyloxy-N-azetidine-1-carboxamide (14)

To a solution of 3-(3-trifluoromethyl)benzyloxy-l-(diphenylmethyl)azetidine (5) (5.3 mmol) in dichloromethane (15 mL) at 0°C, was added a solution of phosgene (1.75M in toluene, 6.4 mmol). The reaction mixture was stirred at room temperature for 2h, concentrated *in vacuo,* then redissolved in THF (15 mL) and treated with ammonium hydroxide (5 mL), added in one portion, at 0°C. The reaction was stirred vigorously for 15h at room temperature, then water (50 mL) and ethyl acetate (40 mL) were added and the layers were separated. The aqueous layer was extracted with ethyl acetate (2 x 40 mL), dried (MgSO₄) and concentrated *in vacuo.*

The residue was triturated using ethyl acetate (10 mL) to yield (14) as a solid (0.91 g, 63%), mp. 167 °C (ethyl acetate).
Found: C, 52.44; H, 4.72; N, 10.23. C₁₄H₁₇CIN₂O₂ requires: C, 52.56; H, 4.78; N, 10.21.

### Preparation of 3-(1-(3-trifluoromethylphenyl)ethyloxy)-1-(diphenylmethyl)azetidine (15)

To a solution of a-methyl-3-trifluoromethylbenzyl alcohol (53 mmol), diisopropylethyl amine (105 mmol) in dichloromethane (150 mL) under nitrogen and cooled to 0 °C, was added methane sulfonyl chloride (63.1 mmol) dropwise over 10 min. The reaction was stirred for 15h. Water (200 mL) was added and the resulting mixture stirred for 10min, poured into potassium carbonate (10% wt/wt aqueous solution, 200 mL) and extracted with dichloromethane (3x150 mL). Combined organic extracts were washed with brine (50 mL) once and then dried (Na₂SO₄), filtered and concentrated *in vacuo.* The residue was dissolved in ethyl ether and washed through a pad of silica, eluting with more ether. The filtrate was concentrated *in vacuo.* This material was used directly, as shown below.

A solution of 1-diphenylmethyl-3-azetidinol (42 mmol) in dimethyl formamide (20 mL) was added via pipette, to a suspension of NaH (60% disp.in oil, 50 mmol) in dimethyl formamide (80 mL) at 0°C. The reaction mixture was stirred at room temperature for 15 min, the crude material from above (assumed 53 mmol) was added dropwise as a solution in dimethyl formamide (30 mL) at 0°C and the reaction mixture stirred at room temperature for 2 h. The reaction was poured into water (200 mL) and extracted with ethyl acetate (3 x 50 mL), the extracts were washed with water (200 mL) and brine (50 mL), dried (MgSO₄) and concentrated *in vacuo.* The residue was purified by chromatography (SiO₂; hexane/ethyl acetate 9/1) to yield 3-(1-(3-trifluoromethylphenyl)ethyloxy)-I-(diphenylmethyl)azetidine (15) as a yellow oil (11.2g, yield 65%). The material was used in the next step without further purification.

### Example 9. 3-(1-(3-Trifluoromethylphenyl)ethyloxy)-azetidine-1-carboxamide (16)

This material was prepared from compound (15) using the procedure described for compound (14) (yield 62%) as a crystalline solid, mp. 130.5-131.5°C (diisopropyl ether).
Found: C, 54.24; H, 5.26; N, 9.69. C₁₄H₁₇ClN₂O₂requires: C, 54.17; H, 5.24.; N, 9.71.

The individual enantiomers of Example 9 are prepared using the same overall synthetic method as described for compound 16, but using the chiral alcohols. The R-enantiomer of Example 9 was prepared from the appropriate chiral 1-(3-trifluoromethyl)phenyl ethyl alcohol. The chiral alcohols may be prepared from 3'-trifluoromethyl-acetophenone by stereoselective reduction, for example using borane and a suitable chiral auxiliary or chiral catalyst (see Corey, EJ; Bakshi, RK; Shibata S. J. Amer. Chem. Soc., 1987, 109, 5551-5553 or Pickard, ST and Smith, HE. J Amer. Chem. Soc., 1990, 112, 5741-5747).

### Examples 10 to 43 - See Table 1

These products were prepared using the procedure described for compound (2).

**Table 1**

| Exampleno | Compound No. | Structure | Formula | MWt | mp | Cfound | Hfound | Nfound | Cexp | Hexp | Nexp | Note |
|---|---|---|---|---|---|---|---|---|---|---|---|---|
| 10 | 17 | 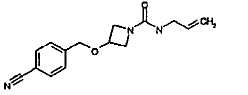 | C15H17N3O2 | 271.32 | 95-96 | 66.69 | 6.29 | 15.32 | 66.40 | 6.32 | 15.48 | |
| 11 | 18 | 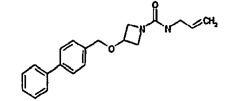 | C20H22N2O2 | 322.41 | 160.0 | 74.52 | 6.87 | 8.61 | 74.51 | 6.88 | 8.68 | |
| 12 | 19 | 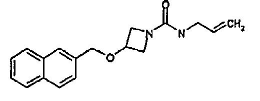 | C18H20N2O2 | 296.37 | 141-142 | 72.96 | 6.77 | 9.65 | 72.95 | 6.80 | 9.45 | |
| 13 | 20 | 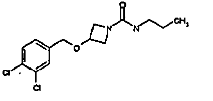 | C14H18Cl2N2O2 | 317.22 | 89-90 | 53.00 | 5.74 | 8.73 | 53.01 | 5.72 | 8.83 | |
| 14 | 21 | 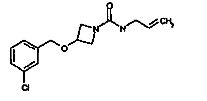 | C14H17CIN2O2 | 280.76 | 67-68 | 59.94 | 6.12 | 9.95 | 59.89 | 6.10 | 9.97 | |
| 15 | 22 | 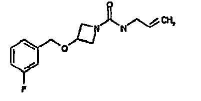 | C14H17FN2O2 | 264.30 | 59-60 | 63.55 | 6.55 | 10.59 | 63.62 | 6.48 | 10.59 | |
| 16 | 23 | 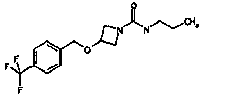 | C15H19F3N2O2 | 316.33 | 128-129 | 56.92 | 6.09 | 8.83 | 56.96 | 6.05 | 8.85 | |
| 17 | 24 | 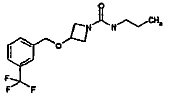 | C15H19F3N2O2 | 316.33 | 62-63 | 56.89 | 6.21 | 8.82 | 56.96 | 6.05 | 8.85 | |
| 18 | 25 | 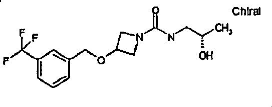 | C15H19F3N2O3 | 332.33 | 67-68 | 54.25 | 5.81 | 8.42 | 54.21 | 5.76 | 8.43 | |
| 19 | 26 | 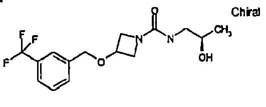 | C15H19F3N2O3 | 332.33 | 67-68 | 54.21 | 5.87 | 8.41 | 54.21 | 5.76 | 8.43 | |
| 20 | 27 | 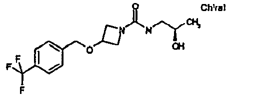 | C15H19F3N2O3 | 332.33 | 97-98 | 54.09 | 5.76 | 8.39 | 54.21 | 5.76 | 8.43 | |
| 21 | 28 | 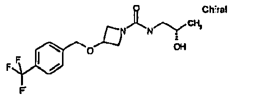 | C15H19F3N2O3 | 332.33 | 97-98 | 54.39 | 5.82 | 8.44 | 54.21 | 5.76 | 8.43 | |
| 22 | 29 | 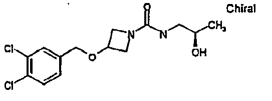 | C14H18CI2N2O3 | 333.22 | 88-89 | 50.46 | 5.34 | 8.39 | 50.46 | 5.44 | 8.40 | |
| 23 | 30 | 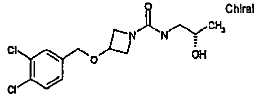 | C14H18CI2N2O3 | 333.22 | 88-89 | 50.49 | 5.36 | 8.61 | 50.46 | 5.44 | 8.40 | |
| 24 | 31 | 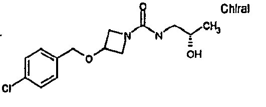 | C14H19CIN2O3 | 298.77 | 85-86 | 56.27 | 6.40 | 9.35 | 56.28 | 6.41 | 9.37 | |
| 25 | 32 | 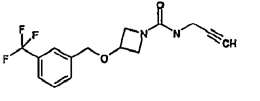 | C15H15F3N2O2 | 312.29 | 90-91 | 57.73 | 4.94 | 8.91 | 57.69 | 4.84 | 8.97 | |
| 26 | 33 | 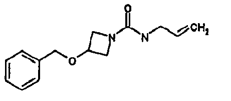 | C14H18N2O2 | 246.31 | 76-77 | 68.29 | 7.35 | 11.37 | 68.27 | 7.37 | 11.37 | |
| 27 | 34 | 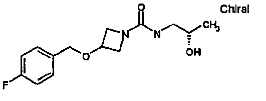 | C14H19FN2O3 | 282.32 | 73-74 | 59.49 | 6.87 | 9.93 | 59.56 | 6.78 | 9.92 | |
| 28 | 35 | 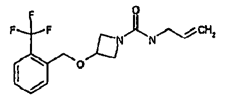 | C15H17F3N2O2 | 314.31 | 63.0 | 57.34 | 5.47 | 8.92 | 57.32 | 5.45 | 8.91 | |
| 29 | 36 | 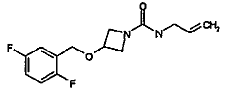 | C14H16F2N2O2 | 282.29 | 75.0 | 59.59 | 5.72 | 9.88 | 59.57 | 5.71 | 9.92 | |
| 30 | 37 | 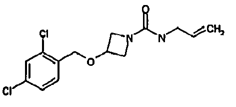 | C14H16CI2N2O2 | 315.20 | 100.0 | 53.15 | 4.99 | 8.86 | 53.35 | 5.12 | 8.88 | |
| 31 | 38 | 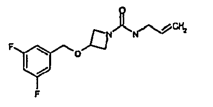 | C14H16F2N2O2 | 282.29 | 79.0 | 59.55 | 5.73 | 9.90 | 59.57 | 5.71 | 9.92 | |
| 32 | 39 | 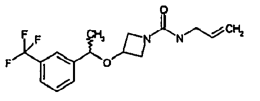 | C16H19F3N2O2 | 328.34 | oil | | | | | | | a |
| 33 | 40 | 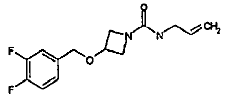 | C14H16F2N2O2 | 282.29 | 82.5-85 | 59.72 | 5.69 | 9.98 | 59.57 | 5.71 | 9.92 | |
| 34 | 41 | 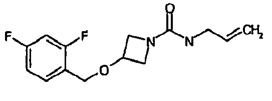 | C14H16F2N2O2 | 282.29 | 91-92.5 | 59.58 | 5.62 | 9.94 | 59.51 | 5.71 | 9.92 | |
| 35 | 42 | 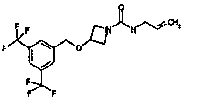 | C16H16F6N2O2 | 382.31 | 80.5-81.5 | 50.38 | 4.25 | 7.32 | 50.27 | 4.22 | 7.32 | |
| 36 | 43 | 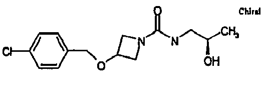 | C14Hl9CIN2O3 | 298.77 | 76-78 | 56.94 | 6.34 | 10.25 | 56.28 | 6.41 | 9.37 | |
| 37 | 44 | 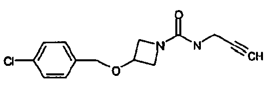 | C14H15CIN2O2 | 278.74 | 123-124 | 60.88 | 5.58 | 9.91 | 60.33 | 5.42 | 10.05 | |
| 38 | 45 | 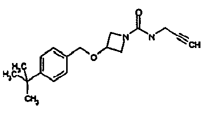 | C18H24N2O2 | 300.40 | 94-96 | 71.89 | 8.08 | 9.28 | 71.97 | 8.05 | 9.32 | |
| 39 | 46 | 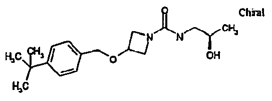 | C18H28N2O3 | 320.44 | oil | | | | | | | b |
| 40 | 47 | 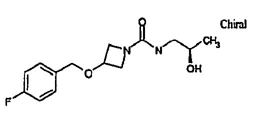 | C14H19FN2O3 | 282.32 | 72-73 | 59.32 | 6.84 | 9.81 | 59.56 | 6.78 | 9.92 | |
| 41 | 48 | 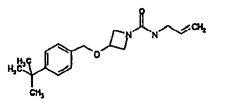 | C18H26N2O2 | 302.42 | 79-80 | 71.25 | 8.79 | 9.36 | 71.49 | 8.67 | 9.26 | |
| 42 | 49 | 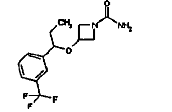 | C14H17F3N2O2 | 302.30 | 110.5-112 | 55.64 | 5.77 | 9.26 | 55.63 | 5.67 | 9.26 | |
| 43 | 50 | 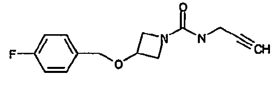 | C14H15FN2O2 | 262.29 | 94-96 | 64.29 | 5.47 | 10.70 | 64.11 | 5.76 | 10.68 | |

| | | | | | | | | | | | | |
|---|---|---|---|---|---|---|---|---|---|---|---|---|
| **Footnotes for Table 1** Footnote a: IR: 3296, 2980, 2943, 2877, 1638, 1545, 1400, 1377, 1330, 1203, 1166, 1127, 1073, 706 cm⁻¹. Footnote b: IR 3319, 2963, 2872, 1634, 1549, 1469, 1403, 1327, 1269, 1184, 1130, 1083, 818 cm⁻¹. | | | | | | | | | | | | |

### Example 44. 3-((3-chlorophenyl)methoxy)-azetidine-l-carboxamide (51)

This material was prepared from compound (1) using the procedure described for compound (14) (yield 87%) as a crystalline solid, m.p. 163-165.5°C (diisopropyl ether).
Found: C, 55.49; H, 5.45; N, 11.40. C₁₁H₁₃ClN₂O₂requires: C, 54.89; H, 5.44.; N, 11.63.

### Assay Procedures

### Binding to CB₁ receptors

The binding of compounds of Formula I to recombinant human CB₁ receptors was determined *in vitro* by standard methods, with reference to the procedure described by Rinaldi-Carmona *et al.* (Rinaldi-Carmona, M., Pialot, F., Congy, C., Redon, E., Barth, F., Bachy, A., Breliere, J. C., Soubre, P., LeFur, G., Life Sci. 1996, 58(15), 1239-1247). Membranes were prepared from HEK293 cells expressing recombinant hCB₁ receptors. Binding assays are performed in a total volume of 250 µL, containing [³H]-SR-141716A (1 nM final concentration), membranes and test compound. Non-specific binding is determined using CP55,940 (10 µM). Serial dilutions are performed starting from test compounds as 10 mM solutions in DMSO. Compounds are tested over the concentration range 10⁻¹⁰ M to 10⁻⁵ M. Kᵢ values are calculated from IC₅₀ values using the Cheng-Prusoff equation.

The thus-determined activity of compounds of formula (I) is shown in Table 2.

**Table 2**

| **Example** | **Kᵢ (HCB₁) nM** |
|---|---|
| Example 6 | 285 |

### Blockade of Δ⁹-THC induced hypolocomotion in mice

The *in vivo* activity of compounds of formula (1) is assayed for ability to antagonise the reduction in locomotor behaviour induced by acute systemic administration of Δ⁹-THC in male C57B1/6 mice. The procedure is as follows.

Test compounds are assessed following acute oral or intraperitoneal administration at a dose of 30 mg/kg. Each study utilises a between-subjects design (typically n=8) and compares the effects of doses of the test agent to those of vehicle and a positive control.

The route of test compound administration, drug volume and injection-test-interval are dependent upon the compounds used. 10 min before testing, a 3 mg/kg dose Δ⁹-THC (or vehicle) is administered to mice by the i.p. route. Automated boxes (AM-1052 activity monitors, Benwick Electronics, Linton Instrumentation) are used to record photocell beam breaks as a measure of locomotor activity. The light beams are arranged on a 7 by 4 matrix on a metal grid. 16 grids are connected in series and Perspex boxes, 20 (width) x 40 (length) x 20 (height) cm, with a flat perforated, Perspex lid are placed in each grid. Mice are placed singly in Perspex boxes and the recording of activity in all 16 boxes starts simultaneously. The mice are left undisturbed to explore the novel activity monitor boxes for 15 minutes while beam breaks are recorded.

Locomotor activity data are subjected to one-way analysis of variance (ANOVA) with drug treatment as a between-subjects factor. A significant main effect is followed up by the performance of Dunnett's test in order to assess which treatment mean(s) are significantly different from the control mean. Significant differences between the vehicle / Δ⁹-THC group and Test compound / Δ⁹-THC groups are assessed by Newman-Keuls test. All statistical analyses were performed using Statistica Software, Version 6.0 (Statsoft Inc.) and Microsoft Excel 7.0 (Microsoft Corp.).

### Regulation of feeding behaviour

The *in vivo* activity of compounds of formula (1) is assayed for ability to regulate feeding behaviour by measuring food consumption in male food-deprived Lister-hooded rats as follows.

Test compounds are assessed following acute administration. Each study utilises a between-subjects design (typically n=8) and compares the effects of doses of the test agent to those of vehicle and a positive control.

The anorectic drug sibutramine, or the reference CB₁ receptor antagonist, SR-141716A, normally serves as a positive control. The route of drug administration, drug volume and injection-test-interval are dependent upon the compounds used. The injection-test-interval is the time between dosing and food re-presentation. Typically, animals are fasted such that at the time of food re-presentation food has been withdrawn for an 18-hour period. Food consumption is assayed at pre-determined time points (typically 1, 2 and 4 hours after administration). Food intake data are subjected to one-way analysis of variance (ANOVA) with drug as a between-subjects factor. A significant main effect is followed up by the performance of Dunnett's test in order to assess which treatment mean(s) are significantly different from the control mean. All statistical analyses were performed using Statistica Software, Version 6.0 (Statsoft Inc.) and Microsoft Excel 7.0 (Microsoft Corp.).

## Claims

1. Use of a compound of formula (I) 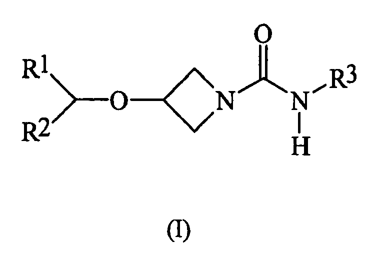 wherein:
R¹ is aryl;
R² is H, alkyl or aryl; and
R³ is hydrogen or alkyl;
or a pharmaceutically acceptable salt thereof, in the manufacture of a medicament for the treatment of a disorder mediated by CD1 receptors, selected from psychosis, schizophrenia, attention deficit disorder, gastrointestinal disorders, smoking cessation, obesity or other eating disorders associated with excessive food intake, and non-insulin dependent diabetes mellitus.

2. A use according to claim 1 wherein R¹ is a substituted or unsubstituted phenyl or naphthyl.

3. A use according to claim 1 or 2 wherein R¹ has 1,2 or 3 substituent groups.

4. A use according to claim 1, 2 or 3 wherein R¹ is chlorophenyl or (trifluoromethyl)phenyl.

5. A use according to claim 1,2,3 or 4 wherein R² is aryl.

6. A use according to any one of claims 1 to 5 wherein R³ is alkyl.

7. A use according to claim 1 wherein the compound is selected from: 3-(bis(4-chlorophenyl)methoxy)-N-(2-propenyl)azetidine-1-carboxamide; and (R)-3-(bis(4-chlorophenyl)methoxy)-N-(2-hydroxypropyl)azetidine-1-carboxamide.

8. A use according to any preceding claim wherein said medicament comprises a pharmaceutically acceptable carrier and as active ingredient an effective amount of a compound of formula (I).

9. A use according to any of claims 1 to 8 wherein the disorder is obesity.

10. A use according to any of claims 1 to 8 for smoking cessation.

11. A use according to any of claims 1 to 8 wherein said disorder is a gastrointestinal disorder.

## Patentansprüche

1. Verwendung einer Verbindung der Formel (I): 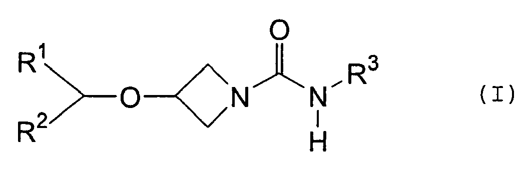 worin
R¹ Aryl ist,
R² H, Alkyl oder Aryl ist und
R³ Wasserstoff oder Alkyl ist;
oder eines pharmazeutisch annehmbaren Salzes davon zur Herstellung eines Medikaments zur Behandlung einer Störung, die durch CB1-Rezeptoren vermittelt werden, ausgewählt aus Psychose, Schizophrenie, Aufmerksamkeitsdefizitstörung, Gastrointestinalstörungen, Raucherentwöhnung, Fettsucht oder anderen Eßstörungen, die mit übermäßiger Nahrungsmittelaufnahme zusammenhängen, und Nicht-Insulin-abhängigem Diabetes mellitus.

2. Verwendung gemäß Anspruch 1, worin R¹ ein substituiertes oder nicht-substituiertes Phenyl oder Naphthyl ist.

3. Verwendung gemäß Anspruch 1 oder 2, worin R¹ 1, 2 oder 3 Substituentengruppen aufweist.

4. Verwendung gemäß Anspruch 1, 2 oder 3, worin R¹ Chlorphenyl oder (Trifluormethyl)phenyl ist.

5. Verwendung gemäß Anspruch 1, 2, 3 oder 4, worin R² Aryl ist.

6. Verwendung gemäß irgendeinem der Ansprüche 1 bis 5, worin R³ Alkyl ist.

7. Verwendung gemäß Anspruch 1, worin die Verbindung ausgewählt ist aus
3-(Bis(4-chlorphenyl)methoxy)-N-(2-propenyl)azetidin-1-carboxamid und
(R)-3-(Bis(4-chlorphenyl)methoxy)-N-(2-hydroxypropyl)-azetidin-1-carboxamid.

8. Verwendung gemäß irgendeinem der vorhergehenden Ansprüche, worin das Medikament einen pharmazeutisch annehmbaren Träger und als wirksamen Inhaltsstoff eine wirksame Menge einer Verbindung der Formel (I) umfaßt.

9. Verwendung gemäß irgendeinem der Ansprüche 1 bis 8, wobei die Störung Fettsucht ist.

10. Verwendung gemäß irgendeinem der Ansprüche 1 bis 8 zur Raucherentwöhnung.

11. Verwendung gemäß irgendeinem der Ansprüche 1 bis 8, worin die Störung eine Gastrointestinalstörung ist.

## Revendications

1. Utilisation d'un composé de formule (I) : 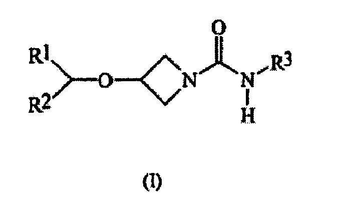 dans laquelle :
R¹ représente un aryle ;
R² représente un atome d'hydrogène, un alkyle ou un aryle ; et
R³ représente un atome d'hydrogène ou un alkyle ;
ou un sel pharmaceutiquement acceptable de celui-ci, dans la fabrication d'un médicament pour le traitement d'un trouble exercé par l'intermédiaire des récepteurs CB₁, choisi parmi la psychose, la schizophrénie, le trouble de déficit de l'attention, les troubles gastro-intestinaux, les troubles engendrés par le fait d'arrêter de fumer du tabac, l'obésité ou d'autres troubles du comportement alimentaire associés à une consommation excessive de nourriture et, le diabète sucré non insulino-dépendant.

2. Utilisation selon la revendication 1, dans laquelle R¹ est un phényle ou un naphtyle substitué ou non substitué.

3. Utilisation selon la revendication 1 ou 2, dans laquelle R¹ comporte 1, 2 ou 3 groupes substituants.

4. Utilisation selon la revendication 1, 2 ou 3, dans laquelle R¹ est un chlorophényle ou un (trifluorométhyl)phényle.

5. Utilisation selon la revendication 1, 2, 3 ou 4, dans laquelle R² est un aryle.

6. Utilisation selon l'une quelconque des revendications 1 à 5, dans laquelle R³ est un alkyle.

7. Utilisation selon la revendication 1, dans laquelle le composé est choisi parmi : le 3-(bis(4-chlorophényl)méthoxy)-N-(2-propényl)azétidine-1-carboxamide ; et le (R)-3-(bis(4-chlorophényl)méthoxy)-N-(2-hydroxypropyl)azétidine-1-carboxamide.

8. Utilisation selon l'une quelconque des revendications précédentes, dans laquelle ledit médicament comprend un véhicule pharmaceutiquement acceptable et, en tant que principe actif, une quantité efficace d'un composé de formule (I).

9. Utilisation selon l'une quelconque des revendications 1 à 8, dans laquelle le trouble est l'obésité.

10. Utilisation selon l'une quelconque des revendications 1 à 8, pour arrêter de fumer du tabac.

11. Utilisation selon l'une quelconque des revendications 1 à 8, dans laquelle ledit trouble est un trouble gastro-intestinal.
